# EUROPÄISCHE PATENTSCHRIFT

(11) **EP 2 291 133 B1**
(45) Veröffentlichungstag und Bekanntmachung des Hinweises auf die Patenterteilung: **03.09.2014**
(21) Anmeldenummer: 09733494.0
(22) Anmeldetag: 23.03.2009
(51) Int. Cl.: A61B 18/14

(54) **BIPOLARE KLEMME FÜR DIE HF-CHIRURGIE**
BIPOLAR CLAMP FOR HF SURGERY
PINCE BIPOLAIRE POUR CHIRURGIE HF

(30) Priorität: 17.04.2008 DE 102008019380
(43) Veröffentlichungstag der Anmeldung: 09.03.2011
(73) Patentinhaber: ERBE Elektromedizin GmbH, 72072 Tübingen (DE)
(72) Erfinder: BELLER, Jürgen, 72810 Gomaringen (DE); BRODBECK, Achim, 72555 Metzingen (DE); HILLER, Jürgen, 72581 Dettingen (DE)
(74) Vertreter: Bohnenberger, Johannes
(86) Internationale Anmeldenummer: PCT/EP2009/002117
(87) Internationale Veröffentlichungsnummer: WO 2009/127314

(56) Entgegenhaltungen:
- WO-A1-2004/082495
- US-A- 6 162 220
- US-A1- 2003 125 729

## Beschreibung

Vorliegende Erfindung betrifft eine bipolare Klemme für die HF-Chirurgie, insbesondere zum Koagulieren von Gewebe, mit wenigstens zwei bipolaren Klemmteilen, die relativ zueinander, einen Fassbereich definierend, zwischen einer Öffnungsstellung und einer Koagulationsstellung bewegbar sind und Elektroden aufweisen zum Koagulieren von durch die Klemmteile im Fassbereich erfasstem Gewebe.

Die Hochfrequenzchirurgie wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydration verändert. Das Gewebe zieht sich dabei derart zusammen, dass die Gefäße verschlossen und Blutungen gestillt werden. Eine darauf evtl. folgende Erhöhung der Stromdichte bewirkt ein explosionsartiges Verdampfen der Gewebeflüssigkeit und ein Aufreißen der Zellmembranen, wobei das Gewebe vollständig durchtrennt wird.

Für die thermische Behandlung biologischen Gewebes werden sowohl bipolare als auch monopolare Techniken angewendet. Bei monopolaren Anordnungen wird der von einem HF-Generator an das elektrochirurgische Instrument zugeführte HF-Strom in das zu behandelnde Gewebe über eine differente Elektrode appliziert, wobei der Strompfad durch den Körper eines Patienten bis zu einer indifferenten Neutralelektrode führt und von dort zurück zum HF-Generator.

Immer mehr an Bedeutung gewinnen jedoch bipolare Instrumente, die mit zwei voneinander elektrisch isolierten Elektrodenteilen ausgebildet sind. Der Stromweg zwischen den Elektrodenteilen ist damit kalkulierbarer und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Solche bipolaren Instrumente werden meist als Klemmzangen ausgebildet, die an ihrem distalen Ende meist zwei Klemmteile umfassen, die über eine zugehörige Betätigungseinrichtung zum Fassen eines Gewebes aufeinander zu bewegt werden können. In einer Klemmstellung oder einer so genannten Koagulationsstellung fließt dann Strom vom einen Klemmteil durch das Gewebe zum anderen Klemmteil, so dass das zwischen den Klemmteilen gefasste Gewebe HF-chirurgisch behandelt werden kann.

Bei bipolaren Klemmen zur Thermofusion bzw. Koagulation wird z.B. in der Hysterektomie auch beim Fassen umfangreicher Gewebevolumina angestrebt, dass die Abgabe der Wärmeenergie zur Koagulation sich im Wesentlichen auf den Bereich zwischen den Fassflächen der Klemmteile beschränkt und somit unerwünschte Erwärmung an anderen Stellen (thermische Lateralschädigung, Erwärmung mit Koagulationswirkung an den Maulteilrücken) minimiert werden. Speziell bei dünnen, kleinvolumigen Strukturen (z.B. Parenchymgewebe, freistehenden Blutgefäßen etc.) ist zusätzlich die Abgabe genau dosierter Wärmeenergien erforderlich, was wiederum nur gewährleistet werden kann, wenn das Gewebevolumen während der Koagulation sicher gefasst bleibt und durch Rekrutierung zusätzlichen Materials, z.B. durch thermischen Schrumpf, nicht nennenswert erhöht wird.

Ein Gerät zur HF-Chirurgie zeigt die EP 0 875 209 B1. Offenbart ist dort eine Klemmzange zur Gewebekoagulation, die an ihrem distalen Ende zwei über eine Gelenkverbindung miteinander verbundene und relativ zueinander bewegbare Klemmteile aufweist. Die beiden Klemmteile begrenzen einen Fassbereich, in dem das zu behandelnde Gewebe festgeklemmt werden kann. Um ein besonders effektives Festlegen des Gewebes im Fassbereich zu erlauben, weist ein Klemmteil bewegbare Fixierungselemente auf, die aus dem Klemmteil aus- und wieder einfahrbar sind. Am anderen Klemmteil sind zwei bipolare Elektroden angeordnet, die voneinander durch eine elektrisch isolierende Schicht getrennt sind. Die erste Elektrode steht dabei vorsprungartig aus diesem Klemmteil hervor, während die zweite Elektrode diese erste Elektrode im Wesentlichen seitlich zurückgesetzt umgibt. Sobald Gewebe zwischen den beiden Klemmteilen gefasst wurde und der HF-Generator zur Koagulationsstromerzeugung aktiviert ist, fließt Strom zwischen der ersten hervorstehenden Elektrode, dem gefassten Gewebe und der zweiten Elektrode, wobei im Gewebe der Koagulationsprozess stattfindet.

Nachteilig hat sich jedoch hier herausgestellt, dass sich das gesamte Klemmteil während der Koagulation erwärmt, was mitunter eine ungewollte Gefäßveränderung, also eine Fremdkoagulation in den Seiten- und Rückenbereichen des Klemmteils zur Folge hat.

Auch die WO 2004-082495 A1 zeigt eine solche bipolare Klemme für die HF-Chirurgie, wobei wieder zwei miteinander zu einem Maulteil verbundene Klemmteile zum Fassen des zu koagulierenden Gewebes vorgesehen sind. Wie schon zuvor, sind an einem Klemmteil die bipolaren Elektroden derart angeordnet, dass nach dem Fassen des Gewebes Strom zwischen der ersten zur zweiten Elektrode fließt. Die Elektroden sind als plattformartige Erhöhungen ausgebildet, wobei die erste Elektrode im Zentrum und die zweite Elektrode als Ringelektrode die erste Elektrode umgebend am Rand der Plattform angeordnet ist. Auch hier hat sich jedoch herausgestellt, dass aufgrund einer reduzierten Wärmeableitung die Erwärmung des die Elektroden tragenden Klemmteils nicht ausgeschlossen werden kann, so dass es auch in Seiten- und dem Rückenbereich des Klemmteils zu einer ungewollten Gewebeveränderung kommt. Zudem ist eine solche Ausbildung nur sehr kostenintensiv realisierbar und aufgrund der engen bipolaren Elektrodenabstände in der Herstellung sehr fehlerintensiv.

Die US 2002/0013583 A1 zeigt ebenfalls eine Klemme für die HF-Chirurgie, bei der unter anderem die bipolaren Elektroden an unterschiedlichen Klemmteilen ausgebildet sind, so dass ein Strom zwischen einer Elektrode des einen Klemmteils und einer anderen Elektrode des anderen Klemmteils fließt. Die Elektroden sind hier auf Leiterbahnen verlegt, die insbesondere auf einander zuweisenden Klemmteilflächen der Klemmteile angeordnet sind. Die Elektroden selber sind als Elektrodenspitzen ausgeführt und derart ausgebildet, dass sie beim Greifen des Gewebes in das zu koagulierende Gewebe eindringen. Dadurch soll erreicht werden, dass sich die die Koagulationsareale durch die jeweiligen bipolaren, in das Gewebe eindringenden Elektroden genauer eingrenzen lassen.

Auch hier hat sich jedoch gezeigt, dass es zu einer Erwärmung des Gewebes in Seiten und dem Rückenbereich der Klemmteile kommt und dass diese Erwärmung Ursache für ungewollte Gewebeveränderungen in diesen Bereichen ist. Zudem birgt die Herstellung solcher Klemmteile produktionstechnische Schwierigkeiten, da die Fixierung der Elektroden auf dem Klemmteil den sehr hohen mechanischen und thermischen Belastungen mitunter nicht gewachsen ist.

Vorliegender Erfindung liegt folglich die Aufgabe zu Grunde, eine bipolare Klemme für die HF-Chirurgie bereit zu stellen, die eine sehr viel präzisere Ausrichtung der Koagulationsareale erlaubt und insbesondere eine nicht gewollte Koagulation von angrenzendem Gewebe verhindert sowie einfacher, fehlerunanfälliger und preiswerter herstellbar ist.

Diese Aufgabe wird durch eine bipolare Klemme und ein Chirurgisches Instrument für die HF-Chirurgie gemäß den Patentansprüchen 1 bzw. 17 gelöst.

Insbesondere wird diese Aufgabe durch eine bipolare Klemme für die HF-Chirurgie, insbesondere zum Koagulieren von Gewebe gelöst, mit wenigstens zwei bipolaren Klemmteilen, die relativ zueinander, einen Fassbereich definierend zwischen einer Öffnungsstellung und einer Koagulationsstellung bewegbar sind und Elektroden aufweisen zum Koagulieren von durch die Klemmteile im Fassbereich erfasstem Gewebe, wobei die Klemmteile im Wesentlichen vollständig aus einem elektrisch leitenden Kern bestehen der von einer elektrisch isolierenden Dünnschicht umgeben ist, wobei die Elektroden als Stiftelektroden auf dem Kern angeordnet sind und die elektrisch isolierende Dünnschicht durchdringend in den Fassbereich hineinragen.

Unter Dünnschicht wird in diesem Zusammenhang eine Schicht verstanden, deren Masse und insbesondere deren Auswirkung aufgrund einer sehr geringen Dicke im direkten Vergleich mit der Masse des Kerns eines jeden Klemmteiles im wesentlichen vernachlässigbar ist. Die genannte Dünnschicht trägt zudem nicht zur Stabilität der Klemmteile bei; sie übernimmt keinerlei Tragfunktion. Aus diesem Grund kann der Kern eines jeden Klemmteils sehr einfach, schnell und kostengünstig, beispielweise über ein Tauchbad oder eine Spritzbehandlung, zur elektrischen Isolation mit einer solchen Dünnschicht versehen werden.

Kern der Erfindung ist es, dass aufgrund der im Wesentlichen vollständig aus einem elektrisch leitenden Kern bestehenden und lediglich von einer elektrisch isolierenden Dünnschicht umgebenden Klemmteile eine Erwärmung dieser Klemmteile aufgrund sehr dünner an den Kernen angeordneter Stiftelektroden vermieden wird. Da die Elektroden als dünne Stifte aus den sehr massiven Kernen der Klemmteile hervorstehen, wird Wärme, die während der Koagulation an den Elektroden entsteht, effektiv abgeleitet, ohne dass es zu einer nennenswerten Erwärmung der Klemmteile kommt.

Wesentlicher Punkt ist, dass die thermische Relaxationszeit, also die Zeit, die eine einzelne Elektrode benötigt, um mit dem Kern und natürlich der Umgebung ins thermische Gleichgewicht zu kommen sehr klein ist.

Neben der Reduktion der Erwärmung der Klemmteile und somit der ungewollten Koagulation von Gewebe, das beispielsweise am Klemmteilrücken anliegt, führt die erfindungsgemäße Anordnung der Elektroden auch zu einer Reduktion des Einflusses der thermischen Schrumpfung auf das Gewebevolumen.

Vorzugsweise ist das Verhältnis der Wärmekapazitäten aller Elektroden zur Wärmekapazität des Kerns derart klein ausgebildet, dass während des Koagulierens ein Erwärmen der Klemmteile im Wesentlichen verhindert wird. Ein solcher Effekt wird auch erreicht, indem die Summe der Volumina der Elektroden sehr viel kleiner ist als die Summe der Volumina der elektrisch leitenden Kerne. Grundsätzlich profitiert ein entsprechend ausgebildetes Klemmteil von einer sehr viel schnelleren Erwärmung der Elektroden vor allem aber von der sehr viel schnelleren Abkühlung, ohne den Kern und somit das Klemmteil zu erhitzen.

Vorzugsweise ist die Summe der Flächen der Elektroden sehr viel kleiner als die Summe der Flächen der Klemmteile gewählt. Das hat neben der indirekt resultierenden verbesserten Wärmeableitung auch zur Folge, dass die Stromdichte an den sehr kleinen Elektrodenflächen im Vergleich zu einer sehr breiten Elektrode, wie sie auch aus dem Stand der Technik bekannt sind, erhöht wird. Dies führt zu einem schnelleren Erhitzen, was die ungewollte Erwärmung des Kerns und des Klemmteils reduziert.

Vorzugsweise weisen die Elektroden an ihrem freien Ende eine abgeflachte Form derart auf, dass deren Eindringen in das gefasste Gewebe in der Koagulationsstellung im Wesentlichen vermieden wird. Die Folge ist eine Reduktion der mechanischen Gewebeschädigung im Fassbereich, wobei trotzdem der Vorteil von dedizierten Koagulationsarealen genutzt werden kann. Die Elektroden sind dazu vorzugsweise als zylindrische Elektroden ausgebildet. Natürlich können aber auch alle anderen Formen und insbesondere rotationssymmetrische Elektrodenformen gewählt werden, so lange das Eindringen in das Gewebe im Wesentlichen vermieden wird. Grundsätzlich ist es bevorzugt, die Elektroden zumindest an ihrer Basis, also dem Bereich mit dem sie am Kern angeordnet sind, orthogonal zu zum Fassbereich weisenden Klemmteilflächen der Klemmteile anzuordnen.

Vorzugsweise sind die Elektroden integral mit dem Kern ausgebildet. Auf diese Weise ist es beispielsweise möglich, den Kern samt Elektroden in einem einzigen Arbeitsgang herzustellen und so die Herstellungskosten zu senken. Zudem garantiert diese Herstellung einen sehr stabilen und widerstandsfähigen Elektrodensitz und führt so zu einem sehr robusten Klemmteil.

Es ist jedoch auch möglich, die Elektroden in komplementären Elektrodenaufnahmen am Kern anzuordnen, so dass sie beispielsweise nach dem Herstellen des Kerns eingesetzt und an besondere Erfordernisse adaptierbar sind. Hier ist auch die Ausbildung einzelner solcher "adäptierbarer" Elektroden möglich. Zudem können in entsprechende Elektrodenaufnahmen auch Distanzhalter, Klemmelemente, mechanische Schneifortsätze etc., isoliert und unisoliert, eingesetzt werden.

Vorzugsweise sind die Elektroden auf den Kernen bzw. den Klemmteilen unter Bildung jeweils eines Zwischenraumes derart voneinander beabstandet und versetzt angeordnet, dass in der Koagulationsstellung die Elektroden eines Klemmteils in die durch die Elektroden des anderen Klemmteils gebildeten Zwischenräume wenigstens teilweise eintauchen. In dieser "Sägezahnstellung" fließt folglich in der Koagulationsstellung Strom im Wesentlichen horizontal zu den Klemmteilflächen der Klemmteile, zwischen den relativ zueinander in die entsprechenden Zwischenräumen wenigstens teilweise eingetauchten Elektroden. Auf diese Weise werden insbesondere die durch die jeweiligen bipolaren Elektroden gebildeten Koagulationsareale räumlich begrenzt.

Vorzugsweise sind die Elektroden derart angeordnet, dass in der Koagulationsstellung der ein Koagulationsareal bewirkende Strom zwischen den einen Zwischenraum bildenden Elektroden und einer in diesen Zwischenraum eintauchenden Elektrode fließt. Die Elektroden sind dabei vorzugsweise derart voneinander beabstandet, dass sich diese Koagulationsareale wenigstens teilweise überlappen. Dies trägt entscheidend zu einer räumlich begrenzten, dort aber sehr effektiven Koagulation bei.

Vorzugsweise sind, um eine gleichmäßige Koagulation zu erreichen, die Zwischenräume einer Mehrzahl der einzelnen Elektroden im Wesentlichen identisch. Dies bedeutet, dass vorzugsweise natürlich auch die Abstände zwischen den, die Zwischenräume begrenzenden Elektroden und der in diesen Zwischenraum eintauchenden Elektrode im Wesentlichen identisch sind. Die Elektroden auf den Klemmteilen bzw. Kernen können grundsätzlich in mehreren parallelen oder nicht parallelen Spalten, in mehreren entsprechenden Reihen oder auch willkürlich angeordnet sein.

Vorzugsweise ist die isolierende Dünnschicht als ein Isolationsplättchen auf den Klemmteilflächen des Kerns und als ein Isolationsmantel auf der restlichen Kernoberfläche ausgebildet. Dies bietet die Möglichkeit, insbesondere bei der Verwendung von nachträglich am Klemmteil angeordneter Elektroden, vorgefertigte und bereits durch den Isoliermantel teilisolierte Kerne nach der Montage der Elektroden durch ein Aufschieben des Isolationsplättchens über die hervorstehenden Elektroden vollständig zu isolieren.

Grundsätzlich sind zur Isolation sämtliche aus dem Stand der Technik und insbesondere aus der HF-Chirurgie bekannte elektrisch isolierende und im wesentlichen thermoresistente Materialien verwendbar, solange sie als Dünnbeschichtung aufbringbar sind und den einschlägigen medizinischen Anforderungen genügen.

Vorzugsweise weist die bipolare Klemme Branchen auf, die über eine Gelenkverbindung miteinander verbunden sind und proximale Betätigungseinrichtungen zum Öffnen und Schließen der distal an den Branchen angeordneten Klemmteile aufweisen, wobei dann die Elektroden entsprechend jeweils einer Kreisbahn um die Gelenkverbindung gebogen sind, auf der sie sich beim Öffnen und Schließen der Klemmteile bewegen.

Insbesondere bei der Anordnung der Branchen in Form einer Zange oder Schere, beispielsweise einem scheren- oder zangenartigen Chirurgischen Instrument, bewegen sich die aus dem Kern hervorstehenden Elektroden kreisbahnförmig um die Gelenkverbindung. Gleiten nun die Elektroden des einen Klemmteils in die durch die Elektroden des anderen Klemmteils gebildeten Zwischenräume ein, wird durch die gemäß der Kreisbahn gebogenen einzelnen Elektroden sichergestellt, dass der Abstand der relativ zueinander eintauchenden Elektroden über die gesamte Elektrodenlänge gleich bleibt, wodurch ein gleichmäßiger Stromfluss zwischen den einzelnen Elektroden gewährleistet ist.

Vorzugsweise nimmt die Länge der aus wenigstens einem Kern herausragenden Elektroden insbesondere proportional zum jeweiligen Abstand von der Gelenkverbindung zu. Auf diese Weise wird sichergestellt, dass kurz vor Erreichen der Koagulationsstellung die einzelnen Elektroden über die Länge der Klemmteile hin gesehen, im Wesentlichen alle gleichzeitig in die jeweiligen Zwischenräume eintauchen.

Weitere Ausführungsformen der Erfindung ergeben sich aus den Unteransprüchen.

Im Folgenden wird die Erfindung anhand zweier Ausführungsformen beschrieben, die durch die beiliegenden Zeichnungen näher erläutert sind. Hierbei zeigen:
- Fig. 1 eine isometrische Darstellung eines HF-Chirurgiegeräts mit einer ersten Ausführungsform der erfindungsgemäßen bipolaren Klemme;
- Fig. 2 eine detaillierte Darstellung der bipolaren Klemme gemäß der Ausführungsform nach Fig. 1;
- Fig. 3 einen Querschnitt durch die bipolare Klemme gemäß Fig. 2;
- Fig. 4 einen Längsschnitt durch die bipolare Klemme gemäß Fig. 2; und
- Fig. 5 einen Querschnitt durch eine zweite Ausführungsform einer bipolaren Klemme.

Im Folgenden werden für gleiche und gleich wirkende Bauteile dieselben Bezugsziffern verwendet, wobei zur Unterscheidung bisweilen Hochindizes ihre Verwendung finden.

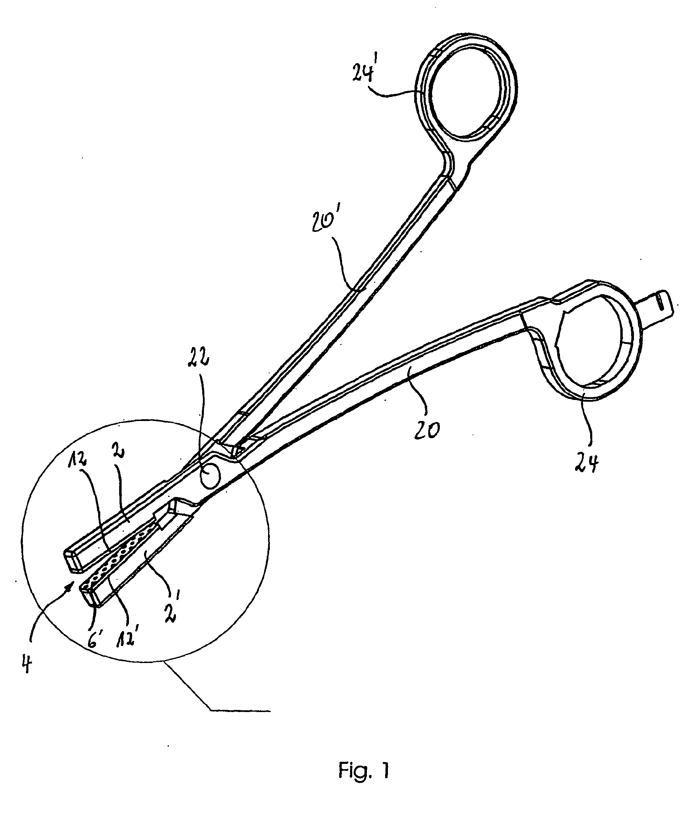

Fig. 1 zeigt eine isometrische Darstellung eines HF-Chirurgieinstruments. Das Chirurgieinstrument umfasst zwei Branchen 20, die über eine Gelenkverbindung 22 in Form einer Zange oder Schere derart miteinander verbunden sind, dass zwei am distalen Ende der Branchen 20 angeordnete Klemmteile 2 von einer Öffnungsstellung in eine Schließstellung und umgekehrt bewegt werden können. Am proximalen Ende der Branchen sind ferner zwei Betätigungsvorrichtungen 24, hier in Form von Griffeinrichtungen vorgesehen, die das Öffnen und Schließen der Branchen 20 und somit auch das Öffnen und Schließen der Klemmteile 2 erlauben.

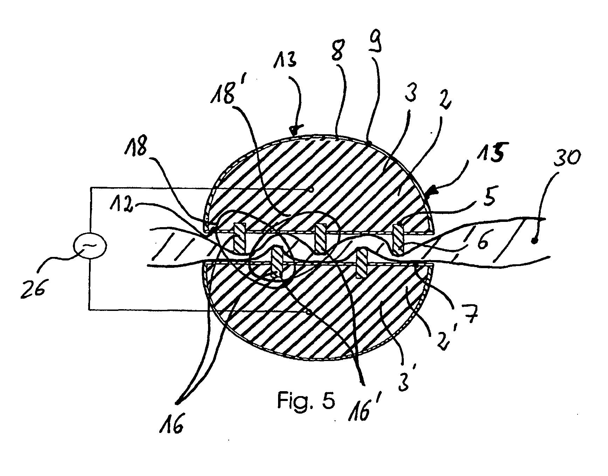

Die Klemmteile 2 bilden zusammen einen Fassbereich 4, in dem zu koagulierendes oder ähnlich HF-chirurgisch zu behandelndes Gewebe (siher Fig. 5) gefasst werden kann. Zur Durchführung der HF-chirurgischen Operation sind an den Klemmteilen 2 und insbesondere an auf den Fassbereich 4 zuweisenden Klemmteilflächen 12 Elektroden 6 angeordnet, die über einen an den Branchen 20 angeschlossenen HF-Generator (nicht dargestellt) auf unterschiedliche Polarität gesetzt werden können. Sobald Gewebe im Fassbereich 4 durch die Klemmteile 2 und insbesondere durch die Elektroden 6 erfasst wird, fließt, nach Aktivierung des HF-Generators, ein Strom zwischen den Klemmteilen 2 bzw. den daran angeordneten Elektroden 6, der zur Koagulation oder ähnlichen HF-chirurgischen Vorgängen verwendet wird.

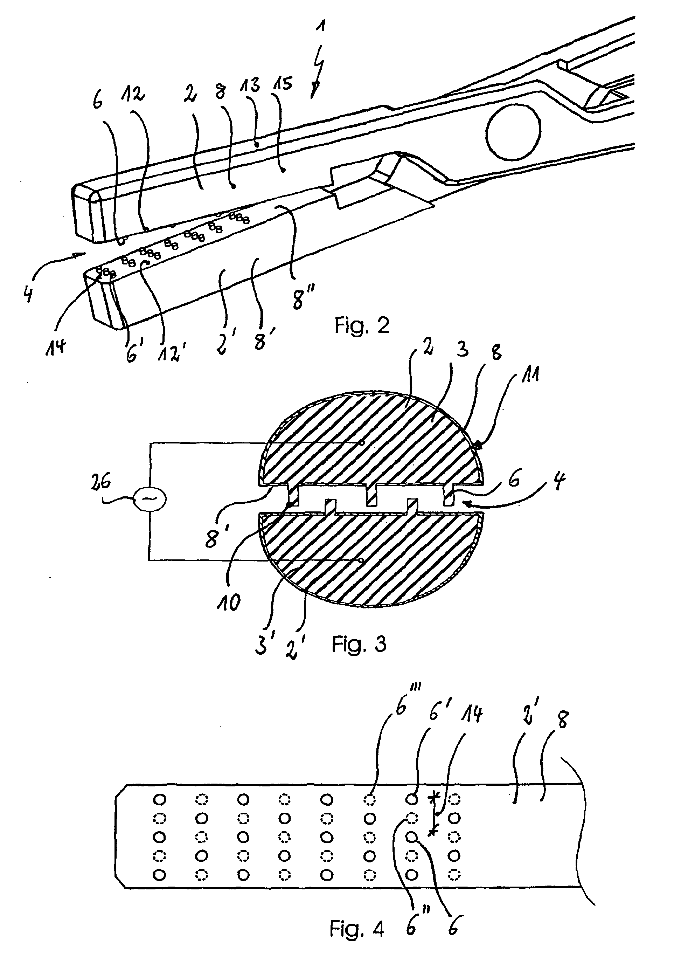

Fig. 2 zeigt die durch die beiden Klemmteile 2 gebildete bipolare Klemme 1 der Ausführungsform aus Fig. 1 in einer isometrischen Detaildarstellung. Deutlich zu erkennen sind die aus den Klemmteilflächen 12 im Wesentlichen orthogonal hervorstehenden Elektroden 6, die als dünne Stiftelektroden zylinderförmig und in jeweils einem bestimmten Abstand 14 zueinander angeordnet sind. Bei dieser Ausführungsform sind die Elektroden 6 entlang der Klemmteile 2 in einem Wechsel von Zweier- und Dreierreihen angeordnet. Natürlich können hier auch andere Elektrodenanordnungen gewählt werden.

Sobald Gewebe im Fassbereich 4 der beiden Klemmteile 2 gefasst ist, wird über die Elektroden 6 Strom in das Gewebe appliziert und die gewünschte HF-chirurgische Operation durchgeführt. Die Klemmteile 2 sind bei dieser Ausführungsform vollständig durch eine elektrisch isolierende Dünnschicht 8 umgeben, so dass ein ungewollter Stromfluss zwischen nicht isolierten Teilen der Klemmteile 2 vermieden wird.

Um das ungewollte Erhitzen, insbesondere der Klemmteilrücken 13 und der Klemmteilseiten 15 zu vermeiden, bestehen die Klemmteile 2 erfindungsgemäß im Wesentlichen vollständig aus einem leitenden Kern 3 (siehe Fig. 3 und 5). Bei dem hier gezeigten Ausführungsbeispiel sind die Elektroden 6 integral mit diesem leitenden Kern 3 ausgebildet (siehe insbesondere Fig. 3). Es wird deutlich, dass die Summe der Wärmekapazitäten der Elektroden 6 eines Klemmteils 2 sehr viel kleiner ist als die Wärmekapazität des Klemmteils 2 bzw. dessen Kerns 3, so dass es bei der Koagulation oder einer dergleichen HF-Operation zu keiner nennenswerten Erwärmung des Klemmteils 2 kommt.

Aufgrund der Größe- und auch Massenunterschiede erhitzen sich die dünnen Elektroden 6 sehr schnell, während sich der Kern 3 aufgrund seiner hohen Masse nur langsam erwärmt.

Selbiges passiert nach dem beenden der HF-Operation: Die im Vergleich zum Kern 3 kleinen Elektroden 6 kühlen sehr schnell wieder auf die Umgebungstemperatur ab; eine ungewollte Koagulation wird vermieden.

Zudem kommt es aufgrund der sehr großen leitenden Fläche des Kerns 3 nicht zu einer durch den Stromfluss indizierten Erwärmung; ausschließlich an den Elektroden 6 steigt die Temperatur rasch an, so dass schnell und räumlich dediziert thermische Behandlungen vorgenommen werden können.

Neben der Reduktion der Erwärmung und somit der ungewollten Koagulation von Gewebe, führt die erfindungsgemäße Ausführung der Klemmteile 2 bzw. des gesamten Chirurgischen Instrumentes auch zu einer Reduktion des Einflusses der thermischen Schrumpfung auf das Gewebevolumen.

Die Elektroden 6 weisen an ihrem freien Ende 10 einen abgeflachten Bereich derart auf, dass sie nicht in das im Fassbereich 4 gefasste Gewebe eindringen. Dies verhindert eine mechanische Beschädigung des Gewebes.

Um eine Koagulation in anderen Bereichen als den Elektrodenbereichen zu vermeiden, ist jeder Kern 3 eines jeden Klemmteils 2 zur elektrischen Isolation von einer isolierenden Dünnschicht 8 umschlossen. Bei dieser Ausführungsform ist diese Dünnschicht 8 vollflächig um den Kern 2 verteilt und insbesondere in Form einer Tauchbeschichtung auf die Kernoberfläche 11 aufgebracht, wobei nur die Elektroden 6 unisoliert aus der Isolationsschicht 8 in den Fassbereich 4 ragen. Hier sei erwähnt, dass es bei der erfindungsgemäßen Ausführung der Klemmteile 2 auch möglich ist auch die Elektroden 6 teilweise mit einer Dünnschicht 8 zu isolieren und beispielsweise nur die freien Enden 10 unisoliert zu lassen.

Schematisch dargestellt ist in Fig. 3 zudem der Anschluss des Hochfrequenzgenerators 26, der derart elektrisch an jedem Kern 3 der Klemmteile 2 angeschlossen ist, dass er diese während der Koagulation auf unterschiedliche Potentiale setzen kann.

Fig. 4 zeigt die Ausführungsform aus Fig. 3 nun in einer Draufsicht bzw. einem Längsschnitt in der Koagulationsstellung, also bei im wesentlichen geschlossenen Klemmteilen 2 und relativ zueinander eingetauchten Elektroden 6. Dargerstellt sind sowohl die Elektroden 6, 6' des Klemmteils 2' als auch die Elektroden 6",6"', die am anderen Klemmteil 2 (siehe Fig. 2) angeordnet sind und hier in die Elektroden-Zwischenräume 14 auf dem Klemmteil 2' eintauchen.

Sichtbar wird dabei die versetzte Anordnung der Elektroden. Alle Elektroden sind auf ihren Klemmteilen 2 so angeordnet, dass zwei Elektroden 6',6 jeweils einen Zwischenraum 14 bilden, in den eine Elektrode 6" des anderen Klemmteils 2' (siehe insbesondere Fig. 3) eintaucht.

Bei dieser Ausführungsform sind die Elektroden so angeordnet, dass der Abstand zwischen der eingreifenden Elektrode 6" und den beiden den Zwischenraum 14 begrenzenden Elektroden 6, 6' gleich ist. Natürlich ist es an Stelle der gezeigten gleichmäßigen Verteilung der Elektroden auch möglich, die Elektroden reihen- oder spaltenversetzt anzuordnen, so dass beispielsweise eine einragende Elektrode eines Klemmteils gleichzeitig von drei einen Zwischenraum 14 begrenzenden Elektroden des anderen Klemmteils umschlossen wird.

Fig. 5 zeigt eine weitere Ausführungsform der bipolaren Klemmte 2 in einem Querschnitt ähnlich zu Fig. 3. Die Elektroden 6 sind hier jedoch nicht integral mit dem Kern verbunden sondern jeweils in Elektrodenaufnahmen 5, die in den jeweiligen Kernen 3 ausgebildet sind, aufgenommen. Bei dieser Ausführungsform ist es so möglich, die Elektroden entweder in die Elektrodenaufnahmen 5 einzupressen, elektrisch leitend einzukleben oder dergleichen zu fixieren. Anstelle von Elektroden können auch andere Elemente, wie Distanzelemente, Fixierelemente etc. von den Elektrodenaufnahmen 5 aufgenommen und am Kern befestigt werden.

Wie auch bei der vorherigen Ausführungsform, wird zwischen den beiden Klemmteilen 2 im Fassbereich 4 das zu koagulierende oder ähnlich HF-chirurgisch zu behandelnde Gewebe 30 gefasst. Sobald Strom über den HF-Generator 26 an die Klemmteile 2 bzw. die Kerne 3 angelegt wird, bilden sich zwischen bipolaren Elektrodenpaaren 16 unterschiedlicher Klemmteile 2 räumlich begrenzte Koagulationsareale 18 aus. Bei der Ausführungsform gemäß Fig. 5 sind die Elektroden 6 dabei so angeordnet, dass sich die jeweils entstehenden Koagulationsareale 18 wenigstens teilweise überlappen.

Bei dieser Ausführungsform ist zudem die isolierende Dünnschicht 8 als eine zweiteilige Dünnschicht ausgeführt. Sie besteht aus einem elektrisch isolierenden Dünnschicht-Isolationsmantel 9, der die Kerne 3 im Seiten- und Rückenbereich 13,15 umgibt, und einem ebenfalls elektrisch isolierend Isolationsplättchen 7, das auf den Klemmteilflächen 12 angeordnet ist und von den Elektroden 6 durchdrungen wird.

### Bezugszeichenliste

- 1: Bipolare Klemme
- 2: Klemmteil
- 3: Kern
- 4: Fassbereich
- 5: Elektrodenaufnahme
- 6: Elektrode
- 7: Isolationsplättchen
- 8: isolierende Dünnschicht
- 9: Isolationsmantel
- 10: freies Ende
- 11: Kernoberfläche
- 12: Klemmteilfläche
- 13: Klemmteilrücken
- 14: Zwischenraum
- 15: Klemmteilseite
- 16: Koagulationspaar
- 18: Koagulationsareal
- 20: Branche
- 22: Gelenkverbindung
- 24: Betätigungseinrichtung
- 26: HF-Generator
- 30: Gewebe

## Patentansprüche

1. Bipolare Klemme für die HF-Chirurgie, insbesondere zum Koagulieren von Gewebe, mit wenigstens zwei bipolaren Klemmteilen (2), die relativ zueinander, einen Fassbereich (4) definierend zwischen einer Öffnungsstellung und einer Koagulationsstellung bewegbar sind und Elektroden (6) aufweisen zum Koagulieren von durch die Klemmteile (2) im Fassbereich (4) erfasstem Gewebe, wobei
die Klemmteile (2) im Wesentlichen vollständig aus einem elektrisch leitenden Kern (3) bestehen, der von einer elektrisch isolierenden Dünnschicht (8) umgeben ist, wobei die Elektroden (6) als Stiftelektroden auf dem Kern (3) angeordnet sind **dadurch gekennzeichnet, dass** die Stiftelektroden die elektrisch isolierende Dünnschicht (8) durchdringend in den Fassbereich (4) hineinragen.

2. Bipolare Klemme nach Anspruch 1,
**dadurch gekennzeichnet, dass**
das Verhältnis der Wärmekapazitäten aller Elektroden (6) zur Wärmekapazität der Kerne (3) derart klein ausgebildet ist, dass während des Koagulierens eine Erwärmung der Klemmteile (2) im Wesentlichen verhindert wird.

3. Bipolare Klemme nach Anspruch 1 oder 2,
**dadurch gekennzeichnet, dass**
die Summe der Volumina der Elektroden (6) kleiner ist als die Summe der Volumina der Kerne (3).

4. Bipolare Klemme nach einem der vorhergehenden Ansprüche,
**dadurch gekennzeichnet, dass**
die Summe der Flächen der Elektroden (6) kleiner ist als die Summe der Flächen der Klemmteile (2).

5. Bipolare Klemme nach einem der vorhergehenden Ansprüche,
**dadurch gekennzeichnet, dass**
die Elektroden (6) an ihrem freien Ende (10) eine abgeflachte Form derart aufweisen, das deren Eindringen in das gefasste Gewebe in der Koagulationsstellung im Wesentlichen vermieden wird.

6. Bipolare Klemme nach einem der vorhergehenden Ansprüche,
**dadurch gekennzeichnet, dass**
die Elektroden (6) eine zylindrische Form aufweisen.

7. Bipolare Klemme nach einem der vorhergehenden Ansprüche,
**dadurch gekenn2eichnet, dass**
die Elektroden (6) integral mit wenigstens einem Kern (3) ausgebildet sind.

8. Bipolare Klemme nach einem Ansprüche 1 bis 6,
**dadurch gekennzeichnet,dass**
die Elektroden (6) in komplementären Elektrodenaufnahmen (5) an wenigstens einem Kern (3) angeordnet sind.

9. Bipolare Klemme nach einem der vorhergehenden Ansprüche,
**dadurch gekennzeichnet, dass**
die Elektroden (6) auf dem Kern (3) orthogonal zu zum Fassbereich (4) weisenden Klemmteilflächen (12) der Klemmteile (2) angeordnet sind.

10. Bipolare Klemme nach einem der vorhergehenden Ansprüche,
**dadurch gekennzeichnet, dass**
die Elektroden (6) auf dem Kern (3) unter Bildung jeweils eines Zwischenraumes (14) derart von einander beabstandet und versetzt angeordnet sind, dass in der Koagulationsstellung die Elektroden (6") eines Klemmteiles (2') in die durch die Elektroden (6, 6') des anderen Klemmteiles (2) gebildeten Zwischenräume (14) wenigstens teilweise eintauchen.

11. Bipolare Klemme nach einem der vorhergehenden Ansprüche, insbesondere Anspruch 10,
**dadurch gekennzeichnet, dass**
die Elektroden (6) derart angeordnet und ausgebildet sind, dass bei gefasstem Gewebe (30) in der Koagulationsstellung ein ein Koagulationsareal (18) bildender Strom zwischen den einen Zwischenraum (14) bildenden Elektroden (6, 6') des einen Klemmteiles (2) und der in diesen Zwischenraum (14) eintauchenden Elektrode (6") des anderen Klemmteiles (2') fließt.

12. Bipolare Klemme nach einem der vorhergehenden Ansprüche, insbesondere Anspruch 10 oder 11,
**dadurch gekennzeichnet, dass**
die Elektroden (6) derart von einander beabstandet sind, dass sich die beim Koagulieren zwischen den jeweils Koagulationspaare (16) bildenden Elektroden (6) entstehenden Koagulationsareale (18) wenigstens teilweise überlappen.

13. Bipolare Klemme nach einem der vorhergehenden Ansprüche, insbesondere Anspruch 10 bis 12,
**dadurch gekennzeichnet, dass**
eine Mehrzahl an Zwischenräumen (14) im Wesentlichen identisch ausgebildet ist.

14. Bipolare Klemme nach einem der vorhergehenden Ansprüche,
**dadurch gekennzeichnet, dass**
die isolierende Dünnschicht (8) als ein Isolationsplättchen (7) auf den Klemmteilflächen (12) und als ein Isolationsmantel (9) auf der restlichen Kernoberfläche (11) ausgebildet ist.

15. Bipolare Klemme nach einem der vorhergehenden Ansprüche,
**gekennzeichnet durch**
Branchen (20), die über eine Gelenkverbindung (22) miteinander verbunden sind und proximale Betätigungseinrichtungen (24) zum Öffnen und Schließen der distal an den Branchen (20) angeordneten Klemmteile (2) aufweisen, wobei die Elektroden (6) entsprechend jeweils einer Kreisbahn um die Gelenkverbindung (22) gebogen sind, auf der sie sich beim Öffnen und Schließen der Klemmteile (2) bewegen.

16. Bipolare Klemme nach Anspruch 15,
**dadurch gekennzeichnet, dass**
die Länge der aus wenigstens einem Kern (3) herausragenden Elektroden (6) proportional zum Abstand zur Gelenkverbindung (22) zunimmt.

17. Chirurgisches Instrument für die HF-Chirurgie, mit
wenigstens einer bipolaren Klemme (1) gemäß einem der vorhergehenden Ansprüche, wenigstens zwei Branchen (20), die über eine Gelenkverbindung (22) miteinander verbunden sind und proximalen Betätigungseinrichtungen (24) zum Öffnen und Schließen der distal an den Branchen (20) angeordneten Klemmteile (2).

## Claims

1. Bipolar clamp for HF surgery, in particular for coagulation of tissue, with at least two bipolar clamping parts (2) which, defining a grasping region (4), are movable relative to each other between an open position and a coagulation position and have electrodes (6) for coagulation of tissue that is grasped in the grasping region (4) by the clamping parts (2), wherein the clamping parts (2) are made substantially completely of an electrically conductive core (3), which is surrounded by an electrically insulating thin layer (8), wherein the electrodes (6) are arranged as pin electrodes on the core (3), **characterized in that** the pin electrodes protrude through the electrically insulating thin layer (8) into the grasping region (4).

2. Bipolar clamp according to Claim 1, **characterized in that** the ratio of the heat capacities of all the electrodes (6) to the heat capacity of the cores (3) is small, in such a way that heating of the clamping parts (2) is substantially prevented during the coagulation.

3. Bipolar clamp according to Claim 1 or 2, **characterized in that** the sum of the volumes of the electrodes (6) is smaller than the sum of the volumes of the cores (3).

4. Bipolar clamp according to one of the preceding claims, **characterized in that** the sum of the surface areas of the electrodes (6) is smaller than the sum of the surface areas of the clamping parts (2).

5. Bipolar clamp according to one of the preceding claims, **characterized in that** the electrodes (6) have a flattened shape at their free end (10), such that penetration of the electrodes into the grasped tissue in the coagulation position is substantially prevented.

6. Bipolar clamp according to one of the preceding claims, **characterized in that** the electrodes (6) have a cylindrical shape.

7. Bipolar clamp according to one of the preceding claims, **characterized in that** the electrodes (6) are formed integrally with at least one core (3).

8. Bipolar clamp according to one of Claims 1 to 6, **characterized in that** the electrodes (6) are arranged in complementary electrode seats (5) on at least one core (3).

9. Bipolar clamp according to one of the preceding claims, **characterized in that** the electrodes (6) on the core (3) are arranged orthogonally with respect to clamping-part surfaces (12) of the clamping parts (2) facing towards the grasping region (4).

10. Bipolar clamp according to one of the preceding claims, **characterized in that** the electrodes (6) on the core (3) are spaced apart from one another and offset, in each case leaving an intermediate space (14), in such a way that, in the coagulation position, the electrodes (6") of one clamping part (2') engage at least partially in the intermediate spaces (14) formed by the electrodes (6, 6') of the other clamping part (2).

11. Bipolar clamp according to one of the preceding claims, in particular Claim 10, **characterized in that** the electrodes (6) are arranged and configured in such a way that, when tissue (30) is grasped in the coagulation position, a current forming a coagulation area (18) flows between the electrodes (6, 6') of one clamping part (2) forming an intermediate space (14) and the electrode (6") of the other clamping part (2') engaging in this intermediate space (14).

12. Bipolar clamp according to one of the preceding claims, in particular Claim 10 or 11, **characterized in that** the electrodes (6) are spaced apart from one another in such a way that the coagulation areas (18) developing between the respective electrodes (6) forming coagulation pairs (16) during the coagulation at least partially overlap.

13. Bipolar clamp according to one of the preceding claims, in particular Claims 10 to 12, **characterized in that** a plurality of substantially identical intermediate spaces (14) is formed.

14. Bipolar clamp according to one of the preceding claims, **characterized in that** the insulating thin layer (8) is configured as an insulation plate (7) on the clamping-part surfaces (12) and as an insulation jacket (9) on the rest of the core surface (11).

15. Bipolar clamp according to one of the preceding claims, **characterized by** arms (20), which are connected to each other by an articulated connection (22) and which have proximal actuating devices (24) for opening and closing the clamping parts (2) arranged distally on the arms (20), wherein the electrodes (6) are each curved about the articulated connection (22) on a circular path on which they move during the opening and closing of the clamping parts (2).

16. Bipolar clamp according to Claim 15, **characterized in that** the length of the electrodes (6) protruding from at least one core (3) increases in proportion to the distance from the articulated connection (22).

17. Surgical instrument for HF surgery, with at least one bipolar clamp (1) according to one of the preceding claims, at least two arms (20), which are connected to each other by an articulated connection (22), and proximal actuating devices (24) for opening and closing the clamping parts (2) arranged distally on the arms (20).

## Revendications

1. Pince bipolaire pour chirurgie HF, en particulier pour la coagulation de tissus, comportant au moins deux pièces de serrage bipolaires (2) qui peuvent se déplacer l'une par rapport à l'autre en définissant une zone de saisie (4) entre une position d'ouverture et une position de coagulation et présentent des électrodes (6) pour la coagulation de tissus tenus par les pièces de serrage (2) dans la zone de saisie (4), les pièces de serrage (2) consistant substantiellement totalement en un noyau conducteur électrique (3) qui est entouré d'une mince couche isolante du point de vue électrique (8), les électrodes (6) sous forme d'électrodes tiges étant disposées sur le noyau (3),
**caractérisée en ce que**
les électrodes tiges rentrent dans la mince couche isolante du point de vue électrique (8) en la traversant dans la zone de saisie (4).

2. Pince bipolaire selon la revendication 1,
**caractérisée en ce que**
le rapport entre les capacités thermiques de toutes les électrodes (6) et la capacité thermique des noyaux (3) est défini assez faible pour empêcher substantiellement un réchauffement des pièces de serrage (2) pendant la coagulation.

3. Pince bipolaire selon la revendication 1 ou 2,
**caractérisée en ce que**
la somme des volumes des électrodes (6) est inférieure à la somme des volumes des noyaux (3).

4. Pince bipolaire selon une des revendications précédentes,
**caractérisée en ce que**
la somme des surfaces des électrodes (6) est inférieure à la somme des surfaces des pièces de serrage (2).

5. Pince bipolaire selon une des revendications précédentes,
**caractérisée en ce que**
les électrodes (6) présentent à leur extrémité libre (10) une forme aplatie de telle sorte que leur pénétration dans le tissu saisi soit sensiblement évitée en position de coagulation.

6. Pince bipolaire selon une des revendications précédentes,
**caractérisée en ce que**
les électrodes (6) ont une forme cylindrique.

7. Pince bipolaire selon une des revendications précédentes,
**caractérisée en ce que**
les électrodes (6) font partie intégrante d'au moins un noyau (3).

8. Pince bipolaire selon une des revendications 1 à 6,
**caractérisée en ce que**
les électrodes (6) sont disposées dans des porte-électrodes complémentaires (5) sur au moins un noyau (3).

9. Pince bipolaire selon une des revendications précédentes,
**caractérisée en ce que**
les électrodes (6) sont disposées sur le noyau (3) orthogonalement par rapport à des surfaces des pièces de serrage (12) tournées vers la zone de saisie (4) des pièces de serrage (2).

10. Pince bipolaire selon une des revendications précédentes,
**caractérisée en ce que**
les électrodes (6) sont disposées sur le noyau (3) espacées et décalées mutuellement en formant respectivement un intervalle (14) de manière à ce que, en position de coagulation, les électrodes (6") d'une pièce de serrage (2') plongent au moins partiellement dans les intervalles (14) constitués par les électrodes (6, 6') de l'autre pièce de serrage (2).

11. Pince bipolaire selon une des revendications précédentes, en particulier la revendication 10,
**caractérisée en ce que**
les électrodes (6) sont disposées et formées de manière à ce que, le tissu (30) étant saisi en position de coagulation, un courant formant une zone de coagulation (18) circule entre les électrodes (6, 6') formant un intervalle (14) de l'une des pièces de serrage (2) et l'électrode (6" ) plongeant dans cet intervalle (14) de l'autre pièce de serrage (2').

12. Pince bipolaire selon une des revendications précédentes, en particulier la revendication 10 ou 11,
**caractérisée en ce que**
les électrodes (6) sont espacées mutuellement de manière à ce que les zones de coagulation (18) se créant lors de la coagulation entre les électrodes (6) formant respectivement des paires de coagulation (16) se chevauchent au moins partiellement.

13. Pince bipolaire selon une des revendications précédentes, en particulier les revendications 10 à 12,
**caractérisée en ce**
**qu'**une pluralité d'intervalles (14) ont sensiblement une conformation identique.

14. Pince bipolaire selon une des revendications précédentes,
**caractérisée en ce que**
la mince couche isolante (8) se présente sous forme d'une plaquette isolante (7) sur les surfaces des pièces de serrage (12) et d'une chemise isolante (9) sur le reste de la surface du noyau (11).

15. Pince bipolaire selon une des revendications précédentes,
**caractérisée par**
des branches (20) qui sont reliées entre elles par un joint articulé (22) et présentent des dispositifs d'actionnement proximaux (24) pour l'ouverture et la fermeture des pièces de serrage (2) disposées distalement au niveau des branches (20), les électrodes (6) étant courbées respectivement suivant une piste circulaire entourant le joint articulé (22) sur laquelle elles se déplacent lors de l'ouverture et de la fermeture des pièces de serrage (2).

16. Pince bipolaire selon la revendication 15,
**caractérisée en ce que**
la longueur des électrodes (6) dépassant d'au moins un noyau (3) augmente proportionnellement à la distance par rapport au joint articulé (22).

17. Instrument chirurgical pour chirurgie HF, comportant
au moins une pince bipolaire (1) selon une des revendications précédentes, au moins deux branches (20) qui sont reliées entre elles par un joint articulé (22) et des dispositifs d'actionnement proximaux (24) pour l'ouverture et la fermeture des pièces de serrage (2) disposées distalement au niveau des branches (20).
